# Europäisches Patentamt
# European Patent Office
# Office européen des brevets

(19)

(11) Publication number: **0 004 720**
**A1**

(12)

# EUROPEAN PATENT APPLICATION

(21) Application number: **79300437.5**

(22) Date of filing: **20.03.79**

(51) Int. Cl.²: **C 07 C 29/10**
**C 13 K 13/00**

(30) Priority: **30.03.78 US 891711**

(43) Date of publication of application:
**17.10.79 Bulletin 79/21**

(84) Designated Contracting States:
**DE FR GB IT**

(71) Applicant: **ICI Americas Inc.**

**Wilmington Delaware 19899(US)**

(72) Inventor: **Debernardis, Albert Joseph**
**113 Sherbrooke Drive**
**Wilmington Delaware 19808(US)**

(72) Inventor: **Kruse, Walter Max**
**1 Woodbury Court**
**Wilmington Delaware 19803(US)**

(74) Representative: **Jones, Martin et al,**
**Imperial Chemical Industries Limited Legal Department:**
**Patents Thames House North Millbank**
**London SW1P 4QG(GB)**

(54) Production of high mannitol content solutions from glucose.

(57) Mannitol-rich aqueous solutions of sorbitol and mannitol containing about 40-45% mannitol on the dry basis are prepared by epimerizing glucose in aqueous solution to obtain an epimerizate containing approximately 30% mannose and 70% glucose on the dry basis, and then hydrogenating this mixture of mannose and glucose in an alkaline solution at low temperatures (about 60-100°C.). Solid crystalline mannitol can be recovered from this aqueous solution by known crystallization methods.

EP 0 004 720 A1

3071/EP.
0004720

# PRODUCTION OF HIGH MANNITOL CONTENT SOLUTIONS FROM GLUCOSE

## BACKGROUND OF THE INVENTION

The present invention relates to a process for producing mannitol-rich solutions of sorbitol and mannitol. More particularly, this invention relates to a process for producing a mannitol-rich solution of sorbitol and mannitol from glucose.

It is well known that a mixture of sorbitol and mannitol in aqueous solution can be produced by catalytic hydrogenation of invert sugar, which is an approximately equimolar mixture of glucose and fructose. Invert sugar, in turn. is commonly obtained by inversion of sucrose (ordinary sugar). The yield of mannitol is ordinarily about 24-26 per cent by weight, based on total dry solids, when hydrogenation is carried out under neutral or midly acidic conditions, such as those disclosed in U.S. Patent 2,759,024. This yield can be increased by carrying out at least part of the hydrogenation under alkaline conditions, as described in U.S. Patents 3,329,729 and 3,763.246 or by appropriate choice of catalyst, as described in U.S. Patent 3,705,199. U.S. Patent 3,329,729 describes a process in which hydrogenation of invert sugar is carried out successively under neutral, alkaline, and acidic conditions, yielding about 30-36% by weight (dry basis) of mannitol. The neutral and alkaline hydrogenation steps in this process are carried out at 50° to 80°C. U.S Patent 3,763,246 describes a process in which invert sugar is hydrogenated successively under alkaline and acid conditions at temperatures over 100°C. and preferably from about 140° to about 170°C., yielding about 27-31% by weight (dry basis) of mannitol. The reaction product obtained according to the process of U.S. Patent 3,705,199 contains about 28-29% by weight (dry basis) of mannitol. In each case the balance of the reaction product is mostly sorbitol.

Enhanced yields of mannitol under alkaline hydrogenation conditions are due to isomerization of part of the glucose present to fructose and mannose. The proportions of glucose, fructose, and mannose in the reaction mixture will vary depending on the alkaline material and the conditions used, and significant quantities of mannose are not ordinarily obtained. Such isomerization is well known in the art, and is discussed, for example, in U.S. Patents 3,329,729 and 3.763,246 cited *supra*. and in Pigman, "The Carbohydrates : Chemistry, Biochemistry, and Physiology, " Academic Press, New York, 1957, pages 60-69.

Mannitol may be recovered from aqueous solutions containing both sorbitol and mannitol by fractional crystallization, as described for example in U. S. Patent 3,632.656.

Hydrogenation of invert sugar is an attractive commercial route to the production of mannitol when the price of sucrose (ordinary sugar) is low. However, sharp rises and fluctuations in the price of sucrose in recent years have indicated a need for alternative routes.

High costs of mannose and fructose in substantially pure form preclude the economic use of these sugars as starting materials, even though mannose yields essentially pure mannitol and fructose yields a 50:50 mixture of sorbitol and mannitol on catalytic hydrogenation. There is a need for a new process for preparing mannitol which uses an inexpensive starting material and which gives a higher yield of mannitol than present processes.

Catalytic hydrogenation of glucose and invert sugar to mixtures of sorbitol and mannitol in aqueous solution which is made alkaline with lime and optionally also with calcium carbonate is described in Canadian Patent 735,972. A supported nickel catalyst is preferred catalyst. Other references teach that hydrogenation of glucose in the presence of a nickel catalyst yields a mixture of sorbitol and mannitol under strongly alkaline conditions (pH 10 or over; British Patent 1,129.586), while substantially pure sorbitol is obtained under milder alkaline conditions (pH 8.3-8.5; British Patent 1,140,477). Hydrogenation of either glucose or mannose in the presence of a platinum catalyst and considerable alkali yields a mixture of sorbitol and mannitol, according to U.S. Patent 1,990,245. This reference also states, however. that pure sorbitol is obtained when glucose is hydrogenated at a pH from 7 to 12 in the presence of a nickel catalyst. Glucose is hydrogenated substantially quantitatively ot sorbitol under neutral or midly acidic conditions; see U.S. Patents 1,963,999 and 2,280 975.

A process for obtaining sorbitol-mannitol solutions from glucose by first catalytically epimerizing glucose in an acidic aqueous solution to obtain an epimerizate of glucose and mannose, and then catalytically hydrogenating this epimerizate in an acidic aqueous solution to obtain an aqueous solution of sorbitol and mannitol, is described in U.S. Patent 4,029,878. Epimerization according to that process is carried out at elevated temperature in an acidic aqueous solution containing at least 50% solids and preferably about 67-70% solids, using a hexavalent molybdenum catalyst such as molybdenum acid or an anion exchange resin in the molybdate form. Hydrogenation catalysts and conditions for hydrogenating the glucose-mannose epimerizate to a mixture of sorbitol and mannitol in that process are conventional. Ordinarily the epimerizate will contain about 30% of mannose on the dry basis, and the mol percentage of mannitol in the final product is also usually about 30%; i.e., the mol percentage of mannitol in the final product does not differ significantly from the mol percentage of mannose in the epimerizate.

Epimerization of glucose in aqueous solution into a mixture of glucose and mannose in the presence of molybdic acid is also described by Bilik in Chem. Zvesti 26, 183-186 (1972). In Bilik, a 17% (by weight) glucose solution is used, and 25% of the glucose is epimerized to mannose.

The mannose yield obtained in the patent to Kruse is significantly higher than that obtained by Bilik. Also, the mannitol yield is significantly higher than those obtained by catalytic hydrogenation of invert sugar under acid or neutral conditions, which as stated above ordinarily yields about 25% mannitol, remainder sorbitol, on the dry basis.

Conversion of glucóse to a mixture of sorbitol and mannitol via catalytic epimerization of glucose with a molybdic acid catalyst to a mixture of glucose and mannose, enzymatic isomerization of the glucose-mannose mixture to a mixture of glucose, mannose, and fructose, and catalytic hydrogenation of this mixture of sorbitol and mannitol, is described in published Japanese patent application of M. Takemura et al., Publication No. 51-75008. Yields of approximately 40 percent by weight of mannitol on the dry basis are obtained.

## SUMMARY OF THE INVENTION

It has been found, according to the present invention, that a mannitol-rich aqueous solution of sorbitol and mannitol is produced from an aqueous solution of glucose in a process which includes the steps of (1) contacting the aqueous solution with an epimerization catalyst under epimerization conditions to convert part of the glucose to mannose, and (2) hydrogenating the solution with hydrogen in the presence of a hydrogenation catalyst under alkaline hydrogenation conditions, thereby producing a mannitol-rich solution of sorbitol and mannitol in which the mol fraction of mannitol exceeds the mol fraction of mannose in the epimerizate from step (1).

The first step in the process of this invention is to contact glucose in aqueous solution with an epimerization catalyst under epimerization conditions to obtain a mixture of glucose and mannose in aqueous solution. The epimerization catalyst is preferably a hexavalent molybdenum catalyst as described and claimed in Patent 4 029,878 previously cited. Although any of the catalysts, catalyst concentrations, temperatures, and other reaction conditions described in Patent 4,029,878 can be used, it is preferred to epimerize glucose in a 50 to 70% aqueous solution which also contains molybdic acid in an amount of about 0.25 percent by weight, based on the initial weight of glucose, and which has a pH of about 3.5 to 4, at temperatures of about 90°-100°C. and atmospheric pressure for about 3 hours. Mannose yields under these conditions are usually in the range of about 25-32 percent by weight (typically about 30 percent) on the dry basis, and quantities of oligosaccharides (principally disaccharides) may range from a trace to about 2 percent (dry basis), the balance being primarily unconverted glucose. Temperatures above 100°C. result in shorter reaction times but in increased quantities of by-products such as oligosaccharides. The quantities of both mannose and oligosaccharides increase as the temperature is increased within the 90°-100°C. range. To minimize the formation of oligosaccharides, which adversely affect the quality of the final sorbitol-mannitol product, it is desirable to use conditions (e.g. temperatures below 95°C.) which yield epimerizates containing not over the 28 percent by weight (dry basis) of mannose, even though this results in a slight decrease in mannitol yield. The reaction rate decreases at pH above about 4, while pH values below 3.5 and especially below 3 result in increased oligosaccharide formation. Catalyst concentrations greater or less than 0.25 percent and reaction times which are either longer or shorter than 3 hours, can be used; the amount of mannose and disaccharides in the epimerizate are less

sensitive to small changes in catalyst concentration and reaction time than to small changes in temperature.

Alternatively, one may use the procedure described by V. Bilik in Chem. Zvesti, 26, 183-186 (1972) previously cited. Epimerization according to the process of Patent 4,029,878, is preferred becausen first a higher yield of mannose is obtained in that process, and secondly. because the higher solids concentrations used in that process make it unnecessary to handle or to evaporate large quantities of water in subsequent process steps.

The glucose-mannose solution obtained on epimerization is purified in order to remove the molybdenum catalyst and other impurities. This may be done by contacting the solution with a cation exchange resin and an anion exchange resin, either simultaneously in a mixed bed resin or consecutively, and with one or more adsorbents, such as an adsorbent resin, activated carbon, or both. The epimerizate solution is preferably diluted with water to a solids content of about 50-60% prior to treatment. The preferred cation exchange resins are strongly acid resins, particularly sulfonated styrene-divinylbenzene resins such as "Permutit Q-4", made by Permutit Co. (division of Sybron Corp of Paramus, New Jersey). The preferred anion exchange resin is "Amberlite XE-275", a weakly basic macroreticular acrylic resin having tertiary amine functionality, made by Rohm and Haas Company of Philadelphia, Pennsylvania. A preferred treatment procedure comprises treatment of the diluted epimerizate with a decolorizing resin (e.g., "Duolite S-30" resin), a strongly acid cation exchange resin (e.g., "Permutit Q-4" resin), a weakly basic anion exchange resin (e.g., "Amberlite XE-275" resin), a second portion of strongly acid cation exchange resin (e.g., "Permutit Q-4" resin), a second portion of weakly basic anion exchange resin (e.g., "Amberlite XE-275" resin) and activated carbon (e.g., "Darco S-51" carbon) in the order named.

0004720

It is necessary to remove molybdenum since hydrogenation catalysts tend to be sensitive to the presence of molybdenum. The treated solution is about neutral, and may have a slightly lower solids content than the untreated solution (say 50% vs. 55% solids). Omission of any of these treatment steps results in a poorer hydrogenation product. For example, omission of the initial decolorizing resin treatment results in appreciably larger amounts of reducing sugar in the product (e. g. , amount over 0. 1% by weight, dry basis vs. amounts of less than 0. 05% when the decolorizing treatment is used). Use of only one stage each of cation and anion exchange treatment instead of two stages each results in larger amounts of impurities in the product. Omission of the final decolorizing carbon treatment also appears to result in larger amounts of impurities.

The purified glucose-mannose epimerizate is catalytically hydrogenated at low temperatures in an aqueous alkaline solution, yielding a reaction product mixture of sorbitol, mannitol, and minor amounts of impurities. The mannitol content of the product is ordinarily about 35 to about 45% by weight on the dry basis.

The solution undergoing hydrogenation is made alkaline by the presence of an alkaline material. This alkaline material may be a compound or a mixture of comounds which exhibits a basic reaction in aqueous medium. This alkaline material may be added to the solution where necessary after epimerization and purification and before hydrogenation in order to adjust the solution to the desired pH. (The purified epimerizate is usually either nonalkaline or insufficiently alkaline for hydrogenation. Addition of an alkaline material may be omitted where the purified epimerizate is sufficiently alkaline). Basic reacting alkali metal compounds are preferred as alkaline materials.

The preferred alkaline material for producing the alkaline reaction medium is sodium hydroxide. Preferred concentrations of sodium hydroxide are from about 0.5 to 0.8% by weight (dry basis), based on sugar, (dry basis). Expressed another way, this represents about 0.5 to 0.8 grams or about 15 to 10 millimols, of alkali per 100 grams of sugar. More broadly, the amount of sodium hydroxide can range from as low as 0.3% up to about 1.0% or more by weight based on sugar. The optimum sodium hydroxide concentration is about 0.6% by weight, based on sugar.

As the alkali concentration is increased, the reaction rate and the percentage of mannitol in the reaction product increase. However, the percentage of impurities in the reaction product also increases as the alkali concentration in increased. At low sodium hydroxide concentration, i.e., about 0.5% by weight or less, reduction of sugars may be incomplete unless longer than normal reaction times and/or higher than normal reaction temperatures (i.e., above about 80°C. for at least part of the reaction period) are used. At sodium hydroxide concentrations below about 0.3 percent by weight, based on sugar, the percentage of mannitol in the product is ordinarily not substantially different from that obtained in the absence of alkali. Furthermore, reaction times which are too long for practical operation, or temperatures substantially above normal for at least part of the reaction period (which results in formation of impurities) must be used in order to get effective reduction of sugars. Within the preferred range of sodium hydroxide concentrations, greater percentages of mannitol and larger amounts of impurities are observed at 0.8 percent NaOH than at 0.5 percent or 0.6 percent NaOH. Alkali concentrations above about 0.8 percent are not ordinarily preferred. Although good mannitol yields are obtainable at alkali concentrations above 1%, such concentrations are not recommended because large quantities used of by-products are formed unless low reaction temperatures are used.    Concentrations herein refer to feed
concentrations.

The alkali metal hydroxide concentrations used according
to this invention give solutions in which the initial (or feed) pH is
in the range of about 9. 5 to about 11.    Preferred alkali concentrations give initial pH values from about 10 to about 10. 5.
Same change in pH may occur during hydrogenation.

Other strong bases, such as potassium hydroxide,
tetramethyl ammonium hydroxide, and other quaternary ammonium
bases, can be used in place of sodium hydroxide to render the
hydrogenation reaction medium alkaline.

Sodium carbonate, other alkali metal carbonates, and
other basic reacting alkali metal salts, can also be used to make
the reaction medium alkaline.

Calcium hydroxide can be used but is not preferred
because the hydrogenation products obtained using calcium hydroxide
have higher amounts of impurities than the products. obtained when
sodium hydroxide is used.

Solution concentrations of not more than about 70% by
weight of sugar solids are ordinarily used for hydrogenation.
Preferred concentrations are from about 50 to about 60% by
weight of sugar solids.    A solution concentration of about 55% by
weight of sugar solids appears to be optimum for hydrogenation.
The purified epimerizate solution may be diluted with water where
necessary.    Solutions containing more than about 70% by weight
of solids are too viscous for easy handling and result in undesirably
large amount of impurities in the product.    Even at 60% solids,
the amounts of impurities in the product are greater than the
amount obtained in solutions containing 55% solids.    Solution
concentrations less than about 50% by weight ordinarily are not
preferred because the product solutions are too dilute for optimum
crystallization of mannitol from the mixed mannitol-sorbitol
product solution.

Conventional supported nickel catalysts which are known in the art can be used for hydrogenation according to this invention. A preferred catalyst is nickel supported on diatomaceous earth, such as that described in U.S. Patent 3,705,199 cited supra. Other known sugar hydrogenation catalysts, containing either nickel or ruthenium as the active catalyst metal, can be used. Examples of such other catalysts include the supported nickel catalyst described in U.S. Patent 3,329,729 cited supra, and the supported ruthenium catalysts described in U.S. Patent 2,868,847. Raney nickel can also be used as a catalyst, but the amounts of nickel required are larger, and the mannitol yields tend to be lower, than when a supported nickel catalyst is used under the same conditions of pressure, temperature, time, and alkali concentration. Nickel catalysts are ordinarily preferred because of their lower cost and lower sensitivity to trace amounts of molybdenum, compared with ruthenium catalysts. The amount of supported nickel catalyst is preferably from about 0.6 to about 1.0 pound of nickel, most preferably about 0.8 pound of nickel, per 100 pounds of sugar. Product purity improves but mannitol formation decreases slightly as concentration is increased within this range.

Low hydrogenation temperatures, at least about 50°C. and ordinarily from about 60° to about 100°C., are used in the present process. The optimum hydrogenation temperature is about 80°C. except when low alkali concentrations (about 0.5% or less based on sugar) are used. Also, it appears that better mannitol yields are obtained at about 80°C. than at higher temperatures, which is probably due to the fact that the hydrogenation rate of glucose increases more rapidly than the isomerization rate as temperature increases. Temperatures above 80°C. for at least part of the reaction period are necessary when sodium hydroxide concentrations of about 0.5% or less based on sugar are used; otherwise, sugar reduction will be incomplete. Hydrogenation at temperatures below the preferred range gives good mannitol yields with good purity provided a sufficiently long reaction time is used. Reaction times are too long for practical use at temperatures below about 50°C.

Pressures can vary over a wide range. Ordinarily elevated pressures from about 250 psig. up to as high as 3000 psig. hydrogen pressure can be used, with little difference in mannitol yields or by-product formation over this range.

Hydrogenation according to the present invention is ordinarily complete in about one to two hours at 80°C. Residence times of about 2 hours (e. g. , about 1. 5 to about 2. 5 hours) at 80°C. and NaOH concentrations of 0. 5% or higher are preferred. Lower reaction temperatures and lower alkali concentrations require longer residence times.

The alkaline hydrogenation can be followed by an acid hydrogenation, such as that described in U.S. Patent 3,329,729, if desired, but ordinarily such acid hydrogenation is unnecessary and does not enhance the yield of mannitol. Acid hydrogenation ordinarily is used only when the sugar content following alkaline hydrogenation in undesirably high, or when the epimerizate contains appreciable quantities (e. g. , more than about 0. 5 percent by weight) of oligosaccharides. Unreduced sugars may be present after alkaline hydrogenation when low alkali concentrations (e. g. , 0. 3-0. 5 percent by weight NaOH based on sugar) are used.

To some extent there is a trade-off between high mannitol yields and low impurity content in the product. The preferred reaction conditions described above have been chosen with a view to achieving good mannitol yields consistent with acceptable by-product formation, rather than with a view to maximizing mannitol yields.

Upon completion of the hydrogenation reaction, the catalyst is removed by filtration or decantation. The filtrate may then be treated to remove impurities where necessary or desirable.

Mannitol can be recovered from the hydrogenation product solution by means known in the art, as for example by fractional crystallization.

The process may be carried out in any suitable type of apparatus which enables initimate contact of reactants and control of operating conditions. The hydrogenation apparatus must be pressure resistant. The process may be carried out in batch, semi-continuous, or continuous operation. Continuous operation of the hydrogenation step is ordinarily preferred.

It will be noted that the mannitol yields achieved in the present process are considerably higher than those achieved in the process of Patent 4,029,878 cited _supra_. When the glucose-mannose epimerizate is hydrogenated under alkaline conditions according to the present invention, some isomerization of glucose (mainly to fructose) also takes place, so that the weight percentage of mannitol (dry basis) in the final product is appreciably higher than the weight percentage of mannose in the epimerizate, also expressed on the dry basis. Mannitol yields are comparable to those in Takemura, Japanese Patent Publication No. 51-75008, but fewer reaction steps are required. The present process fulfills the need for a new route to mannitol which starts with an inexpensive raw material and which gives a high yield of mannitol with a minimum number of reaction steps.

This invention will now be described further by means of the following examples. The hydrogenation catalyst in these examples is nickel on diatomaceous earth, containing about 20 percent by weight of nickel and prepared as described in U.S. Patent 3,705,199, unless otherwise indicated. All percentages are by weight unless otherwise indicated.

## EXAMPLE 1

An aqueous glucose solution, containing 69% by weight of glucose based on total solution weight, and 0.25% by weight of molybdic acid based on glucose, and having a pH of about 3.7, is heated in an air atmosphere at atmospheric pressure to 95°C., and is maintained at 95°-98°C. for 3 hours. A dark brown epimerized glucose product, or epimerizate, containing about 28% by weight of mannose on the dry basis. balance (dry basis) principally glucose, is obtained.

The epimerizate as obtained is diluted to about 60% solids and is purified by successive treatments with "Duolite S-30" decolorizing resin, "Permutit Q-4" strongly acid cation exchange resin, "Amberlite XE-275" weakly basic anion exchange resin, a second portion of "Permutit Q-4" strongly acid cation exchange resin, a second portion of "Amberlite XE-275" weakly basic anion exchange resin, and finally with "Darco S-51" activated carbon. The purified epimerizate contains about 55% by weight of sugar solids on the dry basis.

To the purified glucose-mannose solution (55% solids; 28% mannose dry basis) are added a reduced supported nickel catalyst (0.8 pound of nickel per 100 pounds of sugar) and sodium hydroxide (0.8 pound per 100 pounds of sugar) in a nitrogen atmosphere. The pH of the resultant slurry (hereinafter "feed pH") is 10.6.

This slurry is continuously fed to a series of 4 reactors at a rate giving a total residence time of 1.9 hours, with a residence time of 0.76 hour in the first reactor and 0.38 hour in each of the other 3 reactors. Hydrogen at a pressure of 2000 psig is contacted with the sugar slurry in each reactor. The temperature in all four reactors is 80°C. The hot reaction product mixture discharged from the last reactor is filtered to separate the catalyst and is then cooled to room temperature.

-14-

The pH of the cooled reaction product mixture prior to catalyst separation is 11.3. The filtrate is treated with cation and anion exchange resins. Analysis of the product shows 39.5% by weight of mannitol, 57.6% of sorbitol, 0.77% by weight of isomers other than sorbitol and mannitol. and 0.37% total sugar including 0.02% reducing sugar, all by weight of the dry basis.

### EXAMPLES 2 and 3

These examples show the effects of catalyst/sugar ratio on mannitol and impurities.

Glucose is epimerized and the epimerizate purified in the same manner as in Example 1

Purified epimerized glucose (55% solids; about 28% mannose dry basis) is hydrogenated in the same manner as in Example 1, except that the percentage of nickel catalyst based on sugar (i. e. pounds of Ni per 100 pounds of sugar) are as shown in Table 1 below. Percentages by weight of mannitol, and isomers of sorbitol and mannitol in the product (both on the dry basis are also shown in Table 1. Data from Example 1 are included for comparison.

### EXAMPLE 1

| Example | % NaOH | % Ni | Feed pH | Mannitol | Isomers |
|---------|--------|------|---------|----------|---------|
| 1 | 0.8 | 0.8 | 10.6 | 39.5 | 0.77 |
| 2 | 0.8 | 0.7 | 10.1 | 40.6 | 1.07 |
| 3 | 0.8 | 1.0 | 10.0 | 39.5 | 0.56 |

An increase in catalyst/sugar ratio results in a slight decrease in mannitol and in decreases in isomers when other parameters, such as temperature and residence time, remain constant. as comparison of Examples 1 to 3 shows. Quantities of other impurities generally increase as quantities of isomers increase, and decrease as quantities of isomers decrease.

0004720

-15-

In the above examples, the overall level of impurities (as well as isomer level) is lowest in Example 3 and highest in Example 2.

EXAMPLES 4 AND 5

These examples show that lower NaOH concentrations than those used in Examples 1-3 can be used with good results.

Glucose is epimerized glucose (55% solids; about 28% mannose dry basis) is hydrogenated as in Example 1, except that NaOH concentration is 0.6 percent, based on sugar, and catalyst concentrations (i.e., Ni/sugar ratios) are as shown in Table II below. Temperatures (80°C.) and residence times (1.9 hour) are the same as in Example 1. The feed pH in Examples 4 and 5 is 9.9. Catalyst concentrations (as percent Ni, based on sugar) and percentages by weight (dry basis) of mannitol, sorbitol and isomers in the product are given in Table II below.

TABLE II

| Example | % Ni | Mannitol | Sorbitol | Isomers |
|---|---|---|---|---|
| 4 | 1.0 | 34.2 | 64.3 | 0.29 |
| 5 | 0.8 | 37.4 | 60.5 | 0.55 |

The yield of mannitol in Example 5 is lower than in most examples according to this invention, but still appreciably higher than the mannitol yield (approximately 28%) which would be obtained under non-alkaline conditions. Quantities of impurities in Example 4 are quite low. The percentage of mannitol is higher in Example 5 than in Example 4. Amounts of impurities in Example 5 are greater than in Example 4 but are nevertheless low. This shows that decreasing the amount of catalyst improves mannitol yields but also increases impurity formation.

Comparison of Examples 4 and 5 with Examples 3 and 1, respectively, shows that lower mannitol yields are obtained at NaOH concentrations of 0.6% than at NaOH concentrations of 0.8% when otherwise identical conditions are used. However,

-16-

impurities contents are lower at 0. 6% NaOH than at 0. 8% NaOH.

## EXAMPLE 6

Glucose is epimerized as in Example 1. The epimerizate solution is purified as in Example 1 except that treatment with activated carbon precedes treatment with the second portions of cation and anion exchange resins. The purified epimerized glucose solution contains about 31 percent by weight of mannose on the dry basis.

A slurry of purified epimerized glucose solution (55% solids) prepared as above, sodium hydroxide (0. 5 percent by weight, based on sugar), and nickel catalyst (0. 8 percent by weight Ni, based on sugar) are continuously fed to the 4-reactor system described in Example 1. Total residence time is 2. 2 hours. Temperatures in the four reactors (all in °C.) are 85°, 85°, 90°, and 90°, respectively. The reaction product is filtered and ion exchange treatment as in Example 1. The reaction product is found to contain 40. 3 percent by weight (dry basis) of mannitol.

## EXAMPLES 7 AND 8

These examples show the effect of residence time. Glucose is epimerized as in Example 1. The epimerized products are purified by treatment successively with "Permutit Q-4" cation exchange resin, "Amberlite XF-275" anion exchange resin, and with "Darco S-51" decolorizing carbon.

The epimerized and purified glucose solutions (55% solids) are hydrogenated in the presence of 1. 0% by weight of nickel catalyst (as Ni, based on sugar) in the 4-reactor system described in Example 1. Residence times (in hours), sodium hydroxide concentrations (based on sugar), and product mannitol yields (in percent by weight, dry basis) are given in Table III. below.

-17-

## TABLE III

| Ex-ample | Res. time | % NaOH | Man-nitol |
|---|---|---|---|
| 7 | 1.56 | 0.85 | 41.0 |
| 8 | 2.2 | 0.8 | 42.5 |

Example 7 shows that shorter residence times than used in Examples 1-6 can be used. Comparison of Example 8 with Example 7 and with Examples 1-3 shows that longer residence time results in slightly increased mannitol yield.

### EXAMPLE 9

Glucose is epimerized and purified as described in Example 1. Hydrogenation is carried out in the same manner as in Example 3 except that the sodium hydroxide solution is continuously fed to the first reactor as a separate stream at the rate of 0.8 lb. of NaOH (dry basis) per 100 lb. of sugar and is mixed with the epimerized glucose/catalyst feed slurry at the inlet to the first reactor. Temperature, catalyst concentration and residence time are the same as in Example 3. Analysis of the product shows 39.0% by weight of mannitol on the dry basis.

Although the results in Example 9 are not substantially different from those in Example 3, the mode of operation described in Example 9 is considered preferable because it eliminates the possibility of prolonged contact of epimerized glucose with sodium hydroxide prior to hydrogenation. Impurities tend to be less, and complete sugar reduction is more easily attained, when the alkali is injected into the sugar/catalyst slurry at the entrance to the first reactor instead of being added earlier as in the preceding examples. The fact that similar results are obtained in both Examples 3 and 9 is attributed to the lapse of only a short time between the preparation and the hydrogenation of the sugar/sodium hydroxide/catalyst slurry.

Examples 10 to 16 illustrate processes in which hydrogenation is carried out in a batch process.

## EXAMPLE 10

Glucose in aqueous solution (about 70% by weight glucose) containing 0.25% by weight based on sugar, of molybdic acid, is heated to 100°C. and maintained at this temperature for 2 hours. The resulting epimerized glucose solution is diluted with water and treated successively with "Dowex 50W x 8" strong acid cation exchange resin and 60% by weight of strong base anion exchange resin), and with "Darco S-51" activated carbon. The treated epimerizate contains about 54% by weight of sugar solids, and has a mannose content of about 29 percent by weight on the dry basis.

About 200 ml. of the above purified epimerized solution and 25 ml. of water are charged to an autoclave under a nitrogen atmosphere. Also charged to the autoclave under nitrogen atmisphere are 5 g of a catalyst containing about 20% by weight of nickel on kieselguhr, and 10 ml of 2 M aqueous sodium hydroxide solution. (These amounts give about 1% by weight of Ni and about 0.8 % by weight of NaOH, dry basis, both based on sugar). The resulting slurry is pressured to 1600 psig. with hydrogen, heated to 80°C., and maintained at this temperature for 2.0 hours. The slurry is allowed to cool to room temperature and the catalyst is separated by filtration The dark brown filtrate is treated with a mixed bed ion exchange resin (i. e. , a bed containing an ion exchange resin and a cation exchange resin) and with "Darco S-51" activated carbon, giving a clear solution containing 40.0% by weight of mannitol on the dry basis.

## EXAMPLE 11

Glucose is epimerized and purified as in Example 10.

The epimerized glucose solutions is hydrogenated as in Example 10 except that the hydrogenation temperature is 100°C. and hydrogenation time is one hour. The product solution contains 44.8 percent by weight (dry basis) of mannitol.

However, appreciable quantities of impurities are formed. This suggests that the reaction temperature is too high for the sodium hydroxide concentration and reaction conditions used.

## EXAMPLES 12 AND 13

Glucose is epimerized and purified as in Example 1.

The epimerized glucose solution is hydrogenated as in Example 10 with the following changes : (1) The amount of 2 M.NaOH solution is 15 ml giving a sodium hydroxide concentration of about 1.1% by weight, based on the weight of sugar solids; (2) Hydrogenation temperatures for alkaline hydrogenation are as shown in Table II below, (3) After alkaline hydrogenation, 3 g. of phosphoric acid dissolved in 10 ml of water is added. the hot sugar/catalyst slurry is heated to 160°C., and hydrogenation is contained at 160°C. for 0.5 hour. Quantities of mannitol in the reaction products are shown in Table IV below.

### TABLE IV

| Example | Temp., °C. | Mannitol |
|---------|------------|----------|
| 12 | 80 | 44.0 |
| 13 | 60 | 40.6 |

Quantities of impurities are appreciably lower in Example 13 than in Example 12. At the high alkali concentrations used in these examples low hydrogenation temperatures are desirable in order to minimize by-product formation, even though the mannitol yields are somewhat lower.

## EXAMPLE 14

Glucose in aqueous solution is epimerized by heating an aqueous solution containing 68 percent by weight of glucose based on total solution weight, and 0.25 percent by weight of molybdic acid based on glucose, to 95°C. and maintaining this solution at this temperature for 3 hours. An epimerized glucose product, or epimerizate, is obtained. The solution is decolorized and deionized by treatment with ion exchange resins and activated carbon as set forth in Example 1.

Purified epimerized glucose solution (55 percent solids; 29 percent mannose dry basis) prepared as above is charged to an autoclave. Sodium hydroxide (0.3 percent, based on sugar) and reduced supported nickel catalyst (0.6 percent by weight as Ni, based on sugar) are added to the sugar solution. The resultant slurry has a pH of 9.6. All materials are charged to the autoclave in a nitrogen atmosphere. The autoclave is pressured with hydrogen at room temperature to a pressure of about 1500 psig., is heated to 85°C. with a resultant pressure rise to approximately 1750 psig., is maintained at 85°C. for 2 hours, and is then heated to 130 °C. and maintained at this temperature for 0.25 hours. The reaction product is filtered to separate the catalyst and is cooled to room temperature. Analysis of the product after filtration shows 42.9 percent by weight (dry basis) of mannitol. Reducing sugar (0.13 percent by weight, dry basis) is slightly higher than is desirable. The reducing sugar level can be lowered by using a slightly higher second stage hydrogenation temperature (e.g., 140° under alkaline conditions, or 160° under acid conditions as illustrated in Examples 11 and 12, instead of 130° as shown here).

## EXAMPLE 15

Glucose in 70% (by weight) aqueous solution containing 0.25% by weight, based on glucose, of molybdic acid, is heated to 95°C. and maintained at this temperature for 3 hours. The epimerized glucose solution is diluted with water to about 60% solids and is treated successively with "Darco S-51" activated carbon, "Duolite S-30" decolorizing resin, "Duolite C-25D" strongly acid cation exchange resin, "Amberlite XE-275" weakly basic anion exchange resin, "Permutit Q" strongly acid cation exchange resin, and with "Amberlite IRA-68" weakly basic anion exchange resin. The purified epimerizate is analyzed and is found to contain about 27% by weight of mannose on the dry basis.

The epimerized glucose solution (200 grams, 55 percent solids), sodium hydroxide (10 ml of 2M solution, giving about 0.8 percent by weight NaOH, based on sugar), Raney nickel catalyst (6 grams), and water (25 ml) are charged to an autoclave under a nitrogen atmosphere. The autoclave is pressured with hydrogen to about 1500-1550 psig., heated to 80°C., maintained at 80°C. for 100 minutes, heated to 140°C., and maintained at 140°C for 20 minutes. The autoclave contents are allowed to cool to room temperature and the catalyst is separated by filtration. The filtrate is treated with a mixed bed ion exchange resin as in Example 10. The filtrate contains 37.4 percent by weight of mannitol and 60.5 percent by weight of sorbitol on the dry basis.

### EXAMPLE 16

Glucose is epimerized and purified as in Example 10.

The hydrogenation procedure of Example 10 (alkaline hydrogenation at 80°C. for 2 hours.) is followed except that :
(1) 1.0 g of dry calcium hydroxide powder (giving 1.0% by weight, dry basis, of calcium hydroxide, based on the weight of sugar) is charged to the autoclave in place of sodium hydroxide solution,
(2) the volume of epimerized glucose solution is 190 ml, and
(3) no additional water, other than that contained in the epimerized glucose solution is charged to the autoclave. The product contains 39.6% by weight (dry basis) of mannitol. Although an acceptable mannitol yield is obtained, the amounts of impurities are appreciably greater when calcium hydroxide is used instead of sodium hydroxide as the alkaline agent for alkaline hydrogenation.

CLAIMS :

1. A process for obtaining a mannitol-rich solution of sorbitol and mannitol from an aqueous solution of glucose which comprises the steps of :

(a) contacting said solution with an epimerization catalyst under epimerization conditions, whereby a portion of the glucose in said solution is converted to mannose; and

(b) hydrogenating said solution with hydrogen in the presence of a hydrogenation catalyst under alkaline hydrogenation conditions, thereby obtaining a mannitol-rich solution in which the mol fraction of mannitol exceeds the mol fraction of mannose in the mixture obtained in step (a).

2. A process according to claim 1 in which said solution in step (b) made alkaline by the presence of a basic reacting alkali metal compound.

3. A process according to claim 2 in which said alkali metal compound is an alkali metal hydroxide.

4. A process according to claim 3 in which said alkali metal hydroxide is sodium hydroxide.

5. A process according to claim 4 in which the concentration of sodium hydroxide is from about 0.3 to about 1% by weight, based on sugar.

6. A process according to claim 2 in which said alkali metal compound is added to said solution after epimerization and prior to hydrogenation.

7. A process according to anyone of claims 1 to 6 in which epimerizate is catalytically hydrogenated in alkaline aqueous solution at a temperature from about 60°C. to about 100°C.

8.      A process according to anyone of claims 1 to 7 in which said glucose is epimerized in an acidic solution in the presence of a hexavalent molybdenum catalyst.

9.      A process according to anyone of claims 1 to 8 in which the mixture of glucose and mannose in step (a) is treated with anion and cation exchange resins and with at least one decolourizing material prior to hydrogenation.

European Patent
Office

**EUROPEAN SEARCH REPORT**

Application number

EP 79 30 0437

## DOCUMENTS CONSIDERED TO BE RELEVANT

| Category | Citation of document with indication, where appropriate, of relevant passages | Relevant to claim |
|---|---|---|
| | $\underline{FR - A - 2\ 335\ 479}$ (TOWA KASEI KOGYO)<br><br>* Examples 1,2,4,5,6; claims 1-7 *<br><br>-- | 1-9 |
| | $\underline{CH - A - 468\ 326}$ (HEFTI)<br>* Claims 1-4; example 1 *<br><br>-- | 1-7 |
| | CHEMICAL ABSTRACTS, vol. 85, 1976, no. 107492u<br>Columbus, Ohio, U.S.A.<br>M. TAKEMURA et al.: "Mannitol from glucose"<br><br>JP - A - 76 75008<br>* Abstract *<br><br>---- | 1-9 |

**CLASSIFICATION OF THE APPLICATION (Int. Cl.²)**

C 07 C 29/10
C 13 K 13/00

**TECHNICAL FIELDS SEARCHED (Int.Cl.²)**

C 07 C 29/10

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 18-06-1979 | DELHOMME |

EPO Form 1503.1 06.78